# EUROPEAN PATENT APPLICATION

(11) **EP 4 632 854 A1**
(43) Date of publication of application: **15.10.2025**
(21) Application number: 23900098.7
(22) Date of filing: 08.12.2023
(51) Int. Cl.: H01M 8/18, H01M 4/86, C01G 53/00

(54) **NAPHTHALENE-TYPE COMPOUND AND PREPARATION METHOD THEREFOR AND USE THEREOF**

(30) Priority: 09.12.2022 CN 202211581988; 09.12.2022 CN 202211582520
(71) Applicant: Dalian Institute Of Chemical Physics, Chinese Academy Of Sciences, Liaoning 116023 (CN)
(72) Inventor: LI, Xianfeng, Dalian, Liaoning 116023 (CN); ZHANG, Changkun, Dalian, Liaoning 116023 (CN); ZHAO, Ziming, Dalian, Liaoning 116023 (CN)
(74) Representative: Bayramoglu et al.
(86) International application number: PCT/CN2023/137677
(87) International publication number: WO 2024/120530

(57) **Abstract**

The present application discloses a naphthalene-type compound, preparation method therefore and use therefore. The naphthalene-type compound has a molecular structure substituted with polyhydroxyl, polybenzylamine, and quaternary ammonium or multiple quaternary ammonium functional groups, and compared with a raw material, the naphthalene-type compound has greatly improved water solubility in an acidic aqueous solution. An electrochemical reaction has low raw material costs and a high reaction yield, is carried out under a normal temperature and pressure condition without adding additional catalysts, and is carried out under air conditions without inert gas protection.

## Description

### TECHNICAL FIELD

The present application relates to the field of electrochemical synthesis, specifically to a naphthalene-type compound and preparation method therefore and use therefore.

### BACKGROUND TECHNOLOGY

Aromatic compounds possess a stable framework and a large conjugated structure, making them promising candidates for applications in aqueous energy storage. However, polycyclic aromatic hydrocarbons are often hydrophobic in nature, and their application in aqueous environments requires hydrophilic modification to improve their solubility. Naphthalene derivatives, with their rigid structure and moderate molecular weight, are promising as active materials for further application in aqueous energy storage. Based on the molecular structure-activity relationship, hydrophilic functional groups are introduced into aromatic compounds through hydrophilic modification to improve their solubility. Common hydrophilic functional groups include sulfonic acid groups, amine groups (primary, secondary, tertiary, quaternary), carboxyl groups, phosphate groups, etc. How to efficiently and simply introduce these hydrophilic groups into naphthalene derivatives is widely studied. Wang et al. used the Friedel-Crafts reaction with oxalyl chloride, followed by cyclization and hydrolysis under basic conditions, to obtain naphthoquinone derivatives containing carboxyl groups, which have a solubility of up to 1.2 mol/L in a 2M KOH solution (ACS Energy Lett. 2018, 3, 2404-2409). Tong et al. prepared bi-naphthalene compounds (bilawsone) using a free radical-induced dimerization method, achieving a solubility of 0.56 mol/L in a pH=14 aqueous solution (ACS Energy Lett. 2019, 4, 1880-1887). Additionally, sulfonic acid group-modified naphthoquinone derivatives exhibited a solubility of 0.9 mol/L in 1 mol/L KOH (Sci. Rep. 2016, 6, 39101). However, there have been few reports on improving the solubility of naphthalene derivatives in acidic environments, and the solubility of naphthalene-based compounds still needs to be further enhanced.

### CONTENT OF THE INVENTION

According to one aspect of the present application, a naphthalene-type compound is provided, which is a water-soluble polysubstituted naphthalene-type compound;
the naphthalene-type compound includes a naphthoquinone compound and/or a naphthol compound;
the naphthoquinone compound has a structure selected from the group consisting of the structures shown in Formula (1), (2), and (3);
the naphthol compound has a structure selected from the group consisting of the structures shown in Formula (4), (5), and (6); 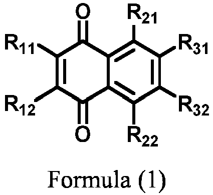 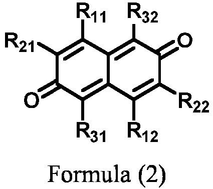 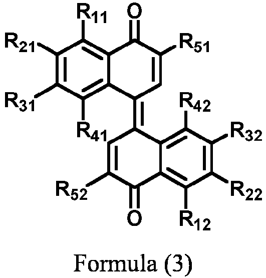 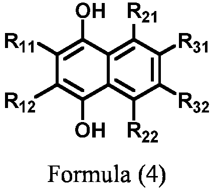 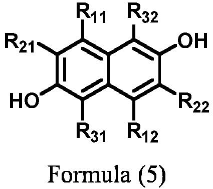 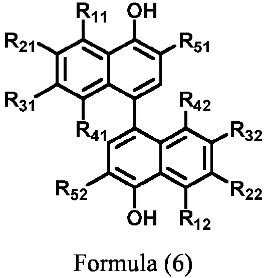
wherein, the naphthalene-type compound having the structure selected from the group consisting of the structures shown in Formula (1), (2), and (3) is an oxidized-state naphthalene-type compound;
the naphthalene-type compound having the structure selected from the group consisted of the structures shown in Formula (4), (5), and (6) is a reduced-state naphthalene-type compound;
the oxidized-state naphthalene-type compound having the structure shown in Formula (1) and the reduced-state naphthalene-type compound having the structure shown in Formula (4) form a redox couple;
the oxidized-state naphthalene-type compound having the structure shown in Formula (2) and the reduced-state naphthalene-type compound having the structure shown in Formula (5) form a redox couple;
the oxidized-state naphthalene-type compound having the structure shown in Formula (3) and the reduced-state naphthalene-type compound having the structure shown in Formula (6) form a redox couple;
R₁₁, R₁₂, R₂₁, R₂₂, R₃₁, R₃₂ are each independently selected from the group consisting of H, F, Cl, Br, I, hydroxyl group, methoxy group, carboxyl group, sulfonic acid group, cyano group, -NR₂, a C₁-C₆ aliphatic group, or a C₁-C₆ aliphatic group substituted with a substituent X-;
the substituent X- in the C₁-C₆ aliphatic group with a substituent X- is selected from the group consisting of carboxyl group, sulfonic acid group, phosphonic acid group, hydroxyl group, methoxy group, or trimethylamino group.

In a second aspect, the present application provides a preparation method of the naphthalene-type compound, including the following steps:
mixing a substrate with an acid, performing electrochemical charging to obtain the naphthoquinone compound, and continuing electrochemical discharging to obtain the naphthol compound.

Optionally, the substrate has a structure shown in Formula (7): 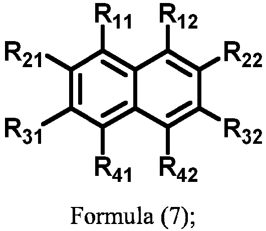
wherein R₁₁, R₂₁, R₃₁, R₄₁, R₁₂, R₂₂, R₃₂, R₄₂ are each independently selected from the group consisting of H, F, Cl, Br, I, hydroxyl group, methoxy group, carboxyl group, sulfonic acid group, cyano group, -NR₂, a C₁-C₆ aliphatic group, or a C₁-C₆ aliphatic group substituted with a substituent X-; and at least one of R₁₁, R₂₁, R₃₁, R₄₁, R₁₂, R₂₂, R₃₂, R₄₂ is a hydroxyl group;
the substituent X- is selected from the group consisting of carboxyl group, sulfonic acid group, phosphonic acid group, hydroxyl group, methoxy group, or trimethylammonio group.

Optionally, the acid includes at least one selected from the group consisting of sulfuric acid, hydrochloric acid, phosphoric acid, acetic acid, citric acid, trifluoroacetic acid, and trifluoromethanesulfonic acid;
the concentration of the acid is in a range from 0.1 mol/L to 5 mol/L; preferably, the acid concentration ranges from 2 to 3 mol/L.

Optionally, the concentration of the acid is any value selected from 0.1 mol/L, 0.5 mol/L, 1 mol/L, 2 mol/L, 3 mol/L, 4 mol/L, 5 mol/L, or a range between any two thereof.

Optionally, the process of electrochemical charging and electrochemical discharging is carried out in an electrochemical reaction cell or an aqueous battery.

Optionally, when the process of electrochemical charging and electrochemical discharging is carried out in the electrochemical reaction cell, a three-electrode system is used with a graphite plate as a working electrode and counter electrode, and a reference electrode selected from the group consisting of a silver-silver chloride electrode, a saturated calomel electrode, and a mercury-mercurous sulfate electrode.
when the process of electrochemical reaction is carried out in the electrochemical reaction cell, a scan rate is in a range from 5 mV s⁻¹ to 500 mV s⁻¹, a voltage range is in a range from 0 V to 1 V;
starting from a raw material, the electrochemical reaction must first undergo electrochemical oxidation to obtain the naphthoquinone compound, followed by electrochemical reduction to obtain the naphthol compound; the sequence cannot be reversed. Therefore, during the reaction process, an electrochemical charging oxidation is performed from low voltage (0 V) to high voltage (1 V), followed by an electrochemical discharging reduction from high voltage (1 V) to low voltage (0 V);
a charging voltage range is in a range from 0.23 to 0.35 V (vs. SCE);
a discharging voltage range is in a range from 0.15 to 0.25 V (vs. SCE).

Optionally, when the process of electrochemical charging and electrochemical discharging is carried out in the aqueous battery, a constant current mode charging and discharging is adopted;
the substrate and acid side serves as a positive electrode, and a negative electrode contains a conjugated redox compound;
the conjugated redox compound is selected from the group consisting of TiO₂⁺/Ti³⁺, V³⁺/V²⁺ and a polyoxometalate (silicotungstic acid);
when the electrochemical reaction process takes place in an aqueous battery, electrochemical oxidation (i.e., charging) is first performed to obtain the naphthoquinone compound, followed by electrochemical reduction (i.e., discharging) to obtain the naphthol compound; the initial state of the conjugated redox compound at the negative electrode should be in the oxidized state;
when the electrochemical reaction process takes place in an aqueous battery, the theoretical capacity of the electrolyte on the negative electrode side must be at least twice the capacity of the product obtained from the electrochemical reaction at the positive electrode;
a current density is in a range from 1 to 120 mA cm⁻²;
a charging cut-off voltage is in a range from 1.2 to 1.4 V;
a discharging cut-off voltage is in a range from 0 to 0.1 V.

Optionally, the preparation process of the water-soluble polysubstituted naphthalene-type compound is as follows:

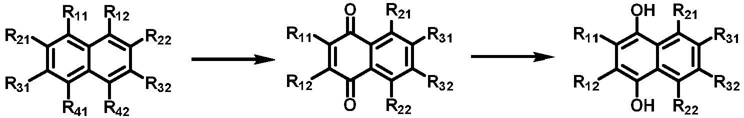

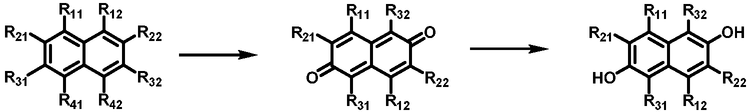

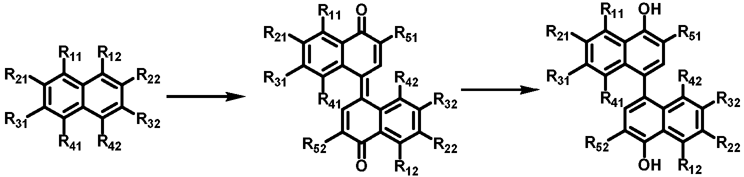

The solubility of the substrate in acidic aqueous solution varies significantly with the type of substituent, and the substrate solution may exist as a clear solution or a turbid suspension containing undissolved solute in a slurry state.

The corresponding naphthoquinone or naphthol compound obtained from the electrochemical reaction has a significantly different structure from that of the substrate, so the solubility is also greatly improved. With the progress of the reaction, it can be observed that part of the turbid suspension becomes a clear solution.

In a third aspect, the present application provides an aqueous redox battery, which includes an electrolyte;
the electrolyte includes a positive electrolyte and a negative electrolyte;
the positive electrolyte contains the naphthoquinone compound of the naphthalene-type compound, and the negative electrolyte contains the naphthol compound of the naphthalene-type compound;
   and/or
the positive electrolyte contains the naphthol compound of the naphthalene-type compound, and the negative electrolyte contains the naphthoquinone compound of the naphthalene-type compound.

In a fourth aspect, the present application provides a flow battery, which is a long-life naphthalene-type liquid flow battery that operates stably in the air;
the flow battery includes an electrolyte;
the electrolyte includes a positive electrolyte and a negative electrolyte;
the electrolyte contains a naphthalene-type compound;
the naphthalene-type compound includes a naphthoquinone compound (oxidized state) and/or a naphthol compound (reduced state);
the positive electrolyte includes the naphthoquinone compound of the naphthalene-type compound, and the negative electrolyte includes the naphthol compound of the naphthalene-type compound;
   or
the positive electrolyte includes the naphthol compound of the naphthalene-type compound, and the negative electrolyte includes the naphthoquinone compound of the naphthalene-type compound;
the concentration of the naphthalene-type compound in the positive electrolyte or negative electrolyte is in a range from 0.01 to 3 mol/L;
wherein the naphthoquinone compound is the naphthoquinone compound of the above naphthalene-type compound or the naphthoquinone compound prepared by the above preparation method;
the naphthol compound is the naphthol compound of the above naphthalene-type compound or the naphthol compound prepared by the above preparation method.

Optionally, the concentration of the naphthalene-type compound in the positive electrolyte or negative electrolyte is in a range from 0.01 mol/L to 1.8 mol/L.

Optionally, the concentration of the naphthalene-type compound in the positive electrolyte or negative electrolyte is in a range from 0.01 mol/L to 1.5 mol/L.

Optionally, the concentration of the naphthalene-type compound in the positive electrolyte or negative electrolyte is in a range from 1.0 mol/L to 1.5 mol/L.

Optionally, the concentration of the naphthalene-type compound in the positive electrolyte or negative electrolyte is any value selected from 0.01 mol/L, 0.05 mol/L, 0.1 mol/L, 0.5 mol/L, 1 mol/L, 1.5 mol/L, or a range between any two thereof.
the naphthoquinone compound has a structure selected from the group consisting of the structures shown in Formula (1), (2), and (3);
the naphthol compound has a structure selected from the group consisting of the structures shown in Formula (4), (5), and (6); 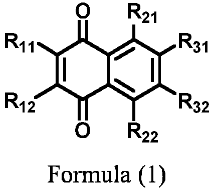 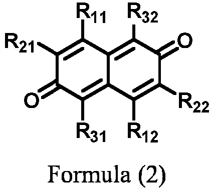 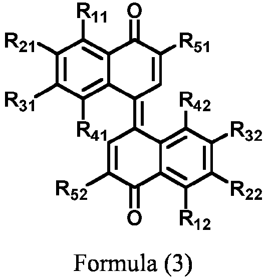 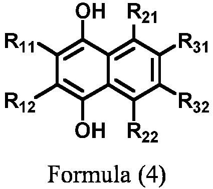 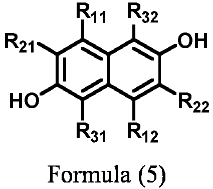 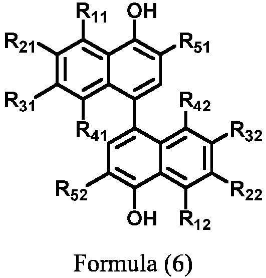
wherein, the naphthalene-type compound having the structure selected from the group consisting of the structures shown in Formula (1), (2), and (3) is an oxidized-state naphthalene-type compound;
the naphthalene-type compound having the structure selected from the group consisted of the structures shown in Formula (4), (5), and (6) is a reduced-state naphthalene-type compound;
the oxidized-state naphthalene-type compound having the structure shown in Formula (1) and the reduced-state naphthalene-type compound having the structure shown in Formula (4) form a redox couple;
the oxidized-state naphthalene-type compound having the structure shown in Formula (2) and the reduced-state naphthalene-type compound having the structure shown in Formula (5) form a redox couple;
the oxidized-state naphthalene-type compound having the structure shown in Formula (3) and the reduced-state naphthalene-type compound having the structure shown in Formula (6) form a redox couple;
R₁₁, R₁₂, R₂₁, R₂₂, R₃₁, R₃₂ are each independently selected from the group consisting of H, F, Cl, Br, I, hydroxyl group, methoxy group, carboxyl group, sulfonic acid group, cyano group, -NR₂, a C₁-C₆ aliphatic group, or a C₁-C₆ aliphatic group substituted with a substituent X-;
the substituent X- in the C₁-C₆ aliphatic group with a substituent X- is selected from the group consisting of carboxyl group, sulfonic acid group, phosphonic acid group, hydroxyl group, methoxy group, or trimethylamino group.

Optionally, the electrochemical reaction equations in the flow battery are as follows:

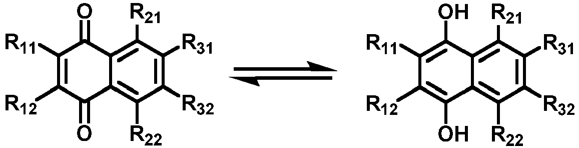

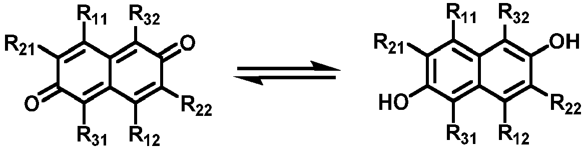

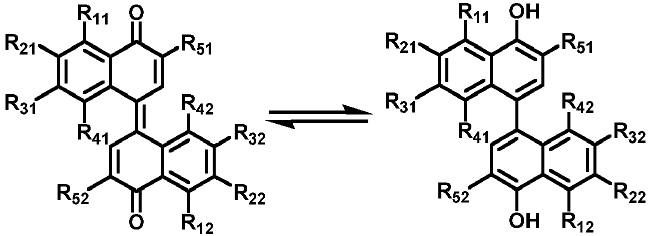

Optionally, the positive electrolyte or negative electrolyte further contains a conjugated redox compound;
the conjugated redox compound is at least one selected from the group consisting of TiO₂⁺/Ti³⁺, V³⁺/V²⁺, and a polyoxometalate;
the polyoxometalate includes silicotungstic acid.

Optionally, the electrolyte further contains an acid;
the acid includes at least one selected from the group consisting of sulfuric acid, hydrochloric acid, phosphoric acid, acetic acid, citric acid, trifluoroacetic acid, and trifluoromethanesulfonic acid;
the concentration of the acid is in a range from 0.01 mol/L to 6 mol/L.

Optionally, the concentration of the acid is any value selected from 0.01 mol/L, 0.05 mol/L, 0.1 mol/L, 0.5 mol/L, 1 mol/L, 2 mol/L, 3 mol/L, 4 mol/L, 5 mol/L, 6 mol/L, or a range between any two thereof.

Optionally, the acid contained in the positive electrolyte and the negative electrolyte has the same concentration as the above-mentioned acid, so as to avoid problems such as volume and concentration asymmetry between the positive and negative electrolytes caused by solvent migration.

Optionally, the naphthalene-type compound is prepared by a method including the following steps:
mixing a substrate with an acidic solution, performing electrochemical charging to obtain the naphthoquinone compound, and continuing electrochemical discharging to obtain the naphthol compound.

The substrate has a structure shown in Formula (7): 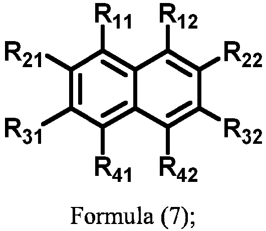
wherein R₁₁, R₂₁, R₃₁, R₄₁, R₁₂, R₂₂, R₃₂, R₄₂ are each independently selected from the group consisting of H, F, Cl, Br, I, hydroxyl group, methoxy group, carboxyl group, sulfonic acid group, cyano group, -NR₂, a C₁-C₆ aliphatic group, or a C₁-C₆ aliphatic group substituted with a substituent X-; and at least one of R₁₁, R₂₁, R₃₁, R₄₁, R₁₂, R₂₂, R₃₂, R₄₂ is a hydroxyl group;
the substituent X- is selected from the group consisting of carboxyl group, sulfonic acid group, phosphonic acid group, hydroxyl group, methoxy group, or trimethylammonio group.

The acidic solution contains at least one selected from the group consisting of sulfuric acid, hydrochloric acid, phosphoric acid, acetic acid, citric acid, trifluoroacetic acid, and trifluoromethanesulfonic acid;
the concentration of the acid solution is in a range from 0.1 mol/L to 5 mol/L.

Optionally, the concentration of the acid is any value selected from 0.1 mol/L, 0.5 mol/L, 1 mol/L, 2 mol/L, 3 mol/L, 4 mol/L, 5 mol/L, or a range between any two thereof.

Optionally, the process of electrochemical charging and electrochemical discharging is carried out in an electrochemical reaction cell or an aqueous battery.

Optionally, when the process of electrochemical charging and electrochemical discharging is carried out in the electrochemical reaction cell,
a three-electrode system is used with a graphite plate as a working electrode and counter electrode, and a reference electrode selected from the group consisting of a silver-silver chloride electrode, a saturated calomel electrode, and a mercury-mercurous sulfate electrode.
when the process of electrochemical reaction is carried out in the electrochemical reaction cell, a scan rate is in a range from 5 mV s⁻¹ to 500 mV s⁻¹, a voltage range is in a range from 0 V to 1 V.

Starting from a raw material, the electrochemical reaction must first undergo electrochemical oxidation to obtain the naphthoquinone compound, followed by electrochemical reduction to obtain the naphthol compound; the sequence cannot be reversed. Therefore, during the reaction process, an electrochemical charging oxidation is performed from low voltage (0 V) to high voltage (1 V), followed by an electrochemical discharging reduction from high voltage (1 V) to low voltage (0 V);
a charging voltage range is in a range from 0.23 to 0.35 V (vs. SCE);
a discharging voltage range is in a range from 0.15 to 0.25 V (vs. SCE).

When the process of electrochemical charging and electrochemical discharging is carried out in the aqueous battery, a constant current mode charging and discharging is adopted;
the substrate and acid side serves as a positive electrode, and a negative electrode contains a conjugated redox compound;
the conjugated redox compound is selected from the group consisting of TiO₂⁺/Ti³⁺, V³⁺/V²⁺ and a polyoxometalate (silicotungstic acid).

When the electrochemical reaction process takes place in an aqueous battery, electrochemical oxidation (i.e., charging) is first performed to obtain the naphthoquinone compound, followed by electrochemical reduction (i.e., discharging) to obtain the naphthol compound; the initial state of the conjugated redox compound at the negative electrode should be in the oxidized state.

When the electrochemical reaction process takes place in an aqueous battery, the theoretical capacity of the electrolyte on the negative electrode side must be at least twice the capacity of the product obtained from the electrochemical reaction at the positive electrode;
a current density is in a range from 1 to 120 mA cm⁻²;
a charging cut-off voltage is in a range from 1.2 to 1.4 V;
a discharging cut-off voltage is in a range from 0 to 0.1 V.

Optionally, the preparation process of the water-soluble polysubstituted naphthalene-type compound is as follows:

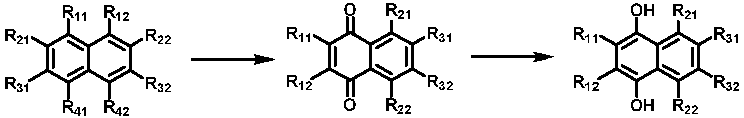

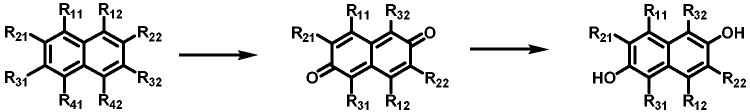

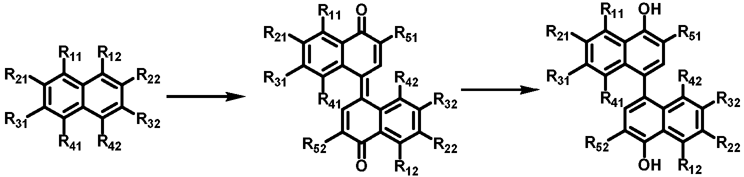

The solubility of the substrate in acidic aqueous solution varies significantly with the type of substituent, and the substrate solution may exist as a clear solution or a turbid suspension containing undissolved solute in a slurry state.

The corresponding naphthoquinone or naphthol compound obtained from the electrochemical reaction has a significantly different structure from that of the substrate, so the solubility is also greatly improved. With the progress of the reaction, it can be observed that part of the turbid suspension becomes a clear solution.

Compared with prior art, the water-soluble polysubstituted naphthalene-type compound provided in the present application by be prepared using the electrochemical method has the following advantages:
1) The electrochemical reaction features low raw material costs, high reaction yields, and can be carried out under normal temperature and pressure without the need for additional catalysts. Unlike other organic redox couples that are prone to oxidation, leading to deactivation, irreversible side reactions, and capacity decay, the compounds of the present applicatio, as organic redox couples, can operate stably in air, exhibiting air insensitivity and long cycle life.
2) The water solubility of the naphthalene-type compound is significantly improved.
3) The naphthalene-type compound has polysubstituted structures, which is difficult to obtain through simple synthetic routes under conventional reaction conditions.
4) In the flow battery, the naphthalene-type compound has low raw material costs, simple preparation processes, and high product yields.
5) The synthesis process of the naphthalene-type compound can be scaled up, making it suitable for large-scale energy storage characteristics of flow batteries.
6) In the flow battery, the naphthalene-type compound exhibits good stability and can undergo charge-discharge cycles in an air environment without significant capacity decay. In contrast, other organic redox couples are prone to oxidation, leading to deactivation, irreversible side reactions, and capacity decay.
7) In the flow battery, the large conjugated and polysubstituted molecular structure of the naphthalene-type compound endow the redox couples with good molecular stability, low probability of side reactions, and resistance to cross-membrane crossover.

### DESCRIPTION OF THE DRAWINGS

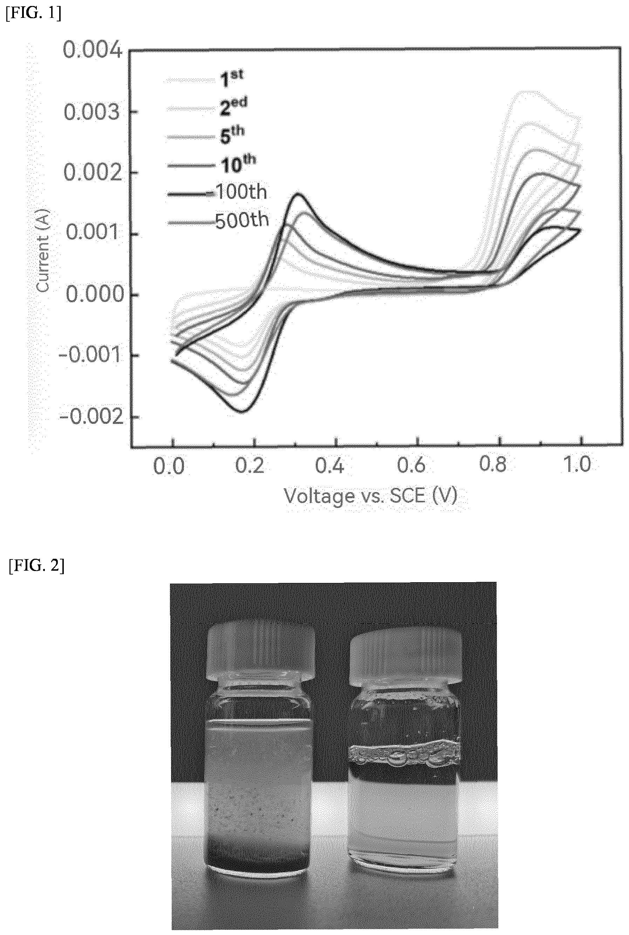

FIG. 1 shows a cyclic voltammetry test curve of Example 1 for preparing 3-benzylamine-2-hydroxy-1,4-dicarbonylnaphthalene (naphthoquinone compound) and 1,2,3-trihydroxy-3-benzylaminonaphthalene (naphthol compound) by using 2,4-dibenzylamine-1-naphthol as raw material through an electrochemical reaction cell. The test conditions are: using graphite as the working electrode and counter electrode, using a saturated calomel electrode as the reference electrode, and the scanning speed is 50 mV/s.
FIG. 2 shows a physical diagram of the positive electrode electrolyte of Example 2 for preparing 3-benzylamine-2-hydroxy-1,4-dicarbonylnaphthalene (naphthoquinone compound) by using 2,4-dibenzylamine-1-naphthol as raw material through an aqueous redox battery. The left suspension is a 3 mol/L sulfuric acid solution of 0.1 mol/L 2,4-dibenzylamine-1-naphthol, and the right clear solution is a 3 mol/L sulfuric acid solution of the electrochemical oxidation product 3-benzylamine-2-hydroxy-1,4-dicarbonylnaphthalene.

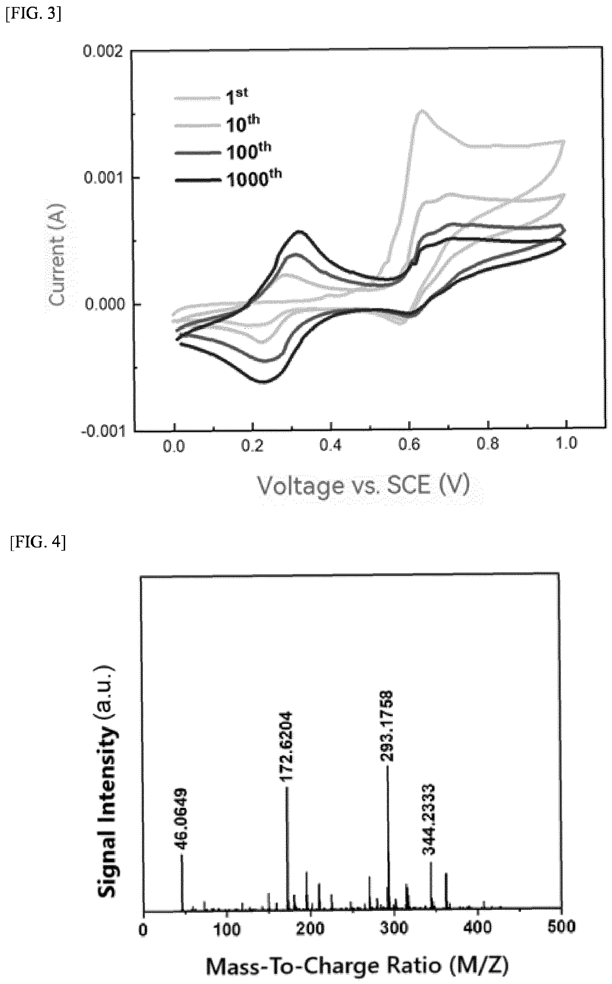

FIG. 3 shows a cyclic voltammetry test curve of Example 3 for preparing 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) by using 2,4,6,8-tetrabenzylamine-1,5-naphthalenediol as raw material through an electrochemical reaction cell. The test conditions are: using graphite as the working electrode and counter electrode, using a saturated calomel electrode as the reference electrode, and the scanning speed is 50 mV/s.
FIG. 4 shows a characteristic molecular weight of the raw material obtained by mass spectrometry analysis in the process of preparing 3,5,7-tribenzylamine-2,8-dihydroxy-1,4-dicarbonylnaphthalene (naphthoquinone compound) by using 2,4,6,8-tetrabenzylamine-1,5-naphthalenediol as raw material through an aqueous redox battery in Example 4.

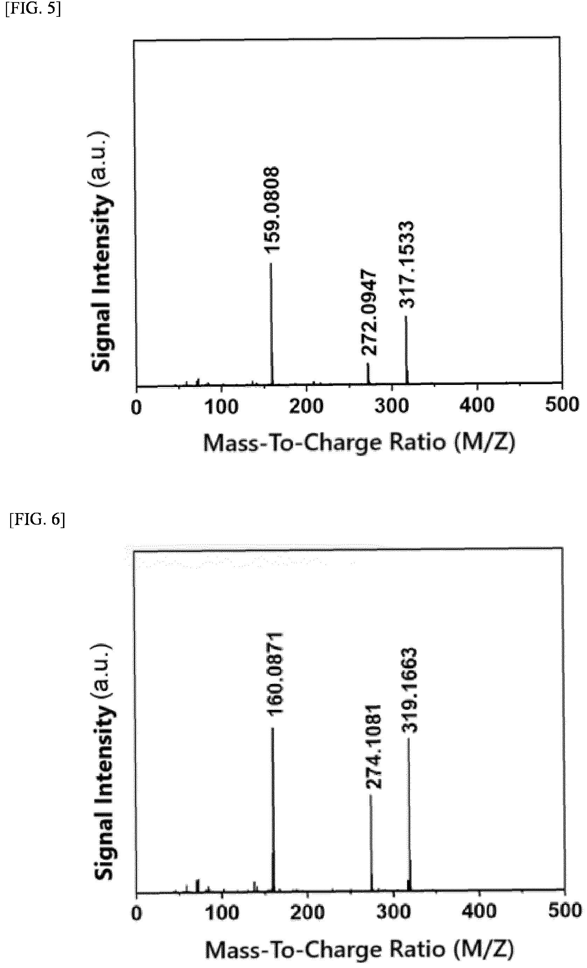

FIG. 5 shows a characteristic molecular weight of the electrochemical oxidation product 3,5,7-tribenzylamine-2,8-dihydroxy-1,4-dicarbonylnaphthalene obtained by mass spectrometry analysis in the process of preparing 3,5,7-tribenzylamine-2,8-dihydroxy-1,4-dicarbonylnaphthalene (naphthoquinone compound) by using 2,4,6,8-tetrabenzylamine-1,5-naphthalenediol as raw material through an aqueous redox battery in Example 4.
FIG. 6 shows a characteristic molecular weight of 1,2,4,8-tetrahydroxy-3,5,7-tribenzylaminonaphthalene obtained by mass spectrometry analysis in the process of preparing 1,2,4,8-tetrahydroxy-3,5,7-tribenzylaminonaphthalene (naphthol compound) by using 2,4,6,8-tetrabenzylamine-1,5-naphthalenediol as raw material through an aqueous redox battery in Example 4.

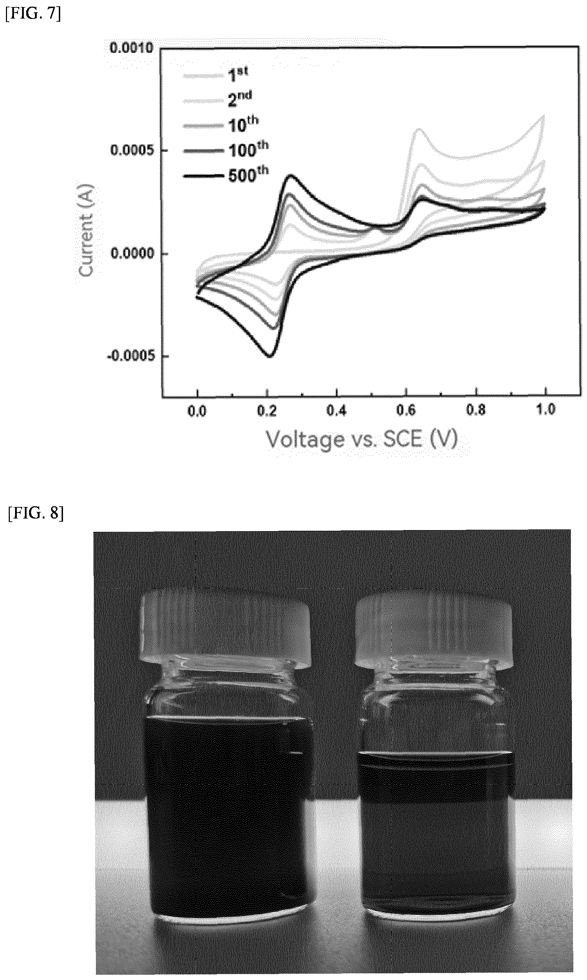

FIG. 7 shows a cyclic voltammetry test curve of Example 5 for preparing 2,2',6,6'-tetrabenzylamine-5,5'-dihydroxy-1,1'-dicarbonylbinaphthalene (naphthoquinone compound) and 1,1',5,5'-tetrahydroxy-2,2',6,6'-tetrabenzylaminonaphthalene (naphthol compound) by using 2,6-dibenzylamine-1,5-naphthalenediol as raw material through an electrochemical reaction cell. The test conditions are: using graphite as the working electrode and counter electrode, using a saturated calomel electrode as the reference electrode, and the scanning speed is 50 mV/s.
FIG. 8 shows a physical diagram of the positive electrode electrolyte of Example 6 for preparing 2,2',6,6'-tetrabenzylamine-5,5'-dihydroxy-1,1'-dicarbonylbinaphthalene (naphthoquinone compound) by using 2,6-dibenzylamine-1,5-naphthalenediol as raw material through an electrochemical reaction cell. The left suspension is a 3 mol/L sulfuric acid solution of 0.1 mol/L 2,6-dibenzylamine-1,5-naphthalenediol, and the right clear solution is a 3 mol/L sulfuric acid solution of the electrochemical oxidation product 2,2',6,6'-tetrabenzylamine-5,5'-dihydroxy-1,1'-dicarbonylbinaphthalene.

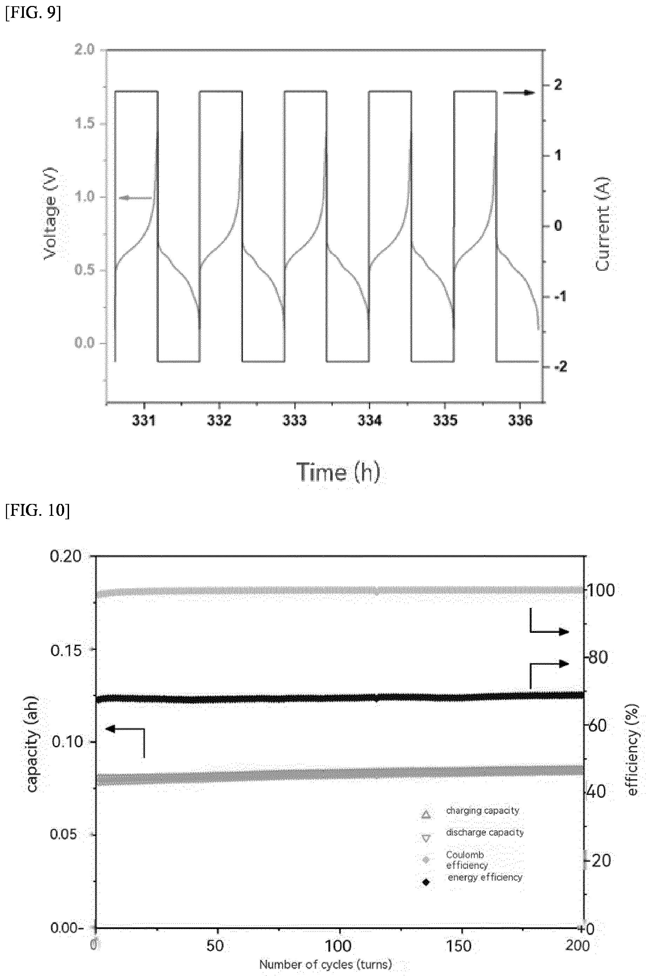

FIG. 9 shows a current-voltage curve of the flow battery cycle stability test using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery. The test conditions are: the current density is 40 mA cm⁻², and the cut-off voltages are 1.4 V and 0.1 V.
FIG. 10 shows a cycle stability curve of the flow battery long-cycle test (under air conditions) using low-concentration 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery. The test conditions are: the current density is 40 mA cm⁻², and the cut-off voltages are 1.4 V and 0.1 V.

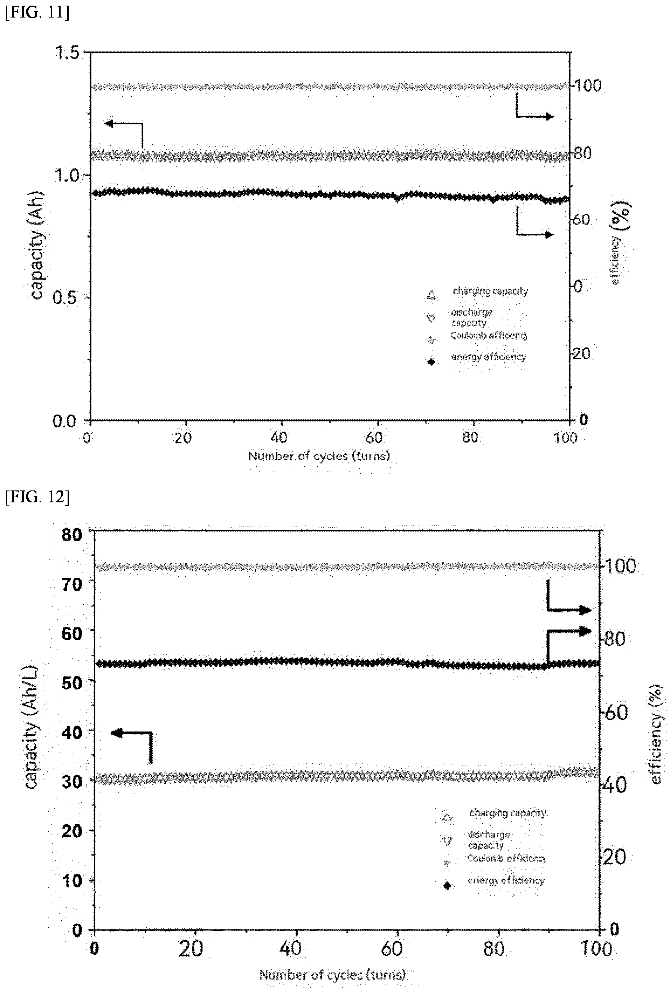

FIG. 11 shows a cycle stability curve of the flow battery long-cycle test (under air conditions) using high-concentration 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery. The test conditions are: the current density is 40 mA cm⁻², and the cut-off voltages are 1.4 V and 0.1 V.
FIG. 12 shows a cycle stability curve of the flow battery long-cycle test (under air conditions) using high-concentration 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery, with the negative electrode equipped with a vanadium electrolyte. The test conditions are: the current density is 40 mA cm⁻², and the cut-off voltages are 1.4 V and 0.1 V.

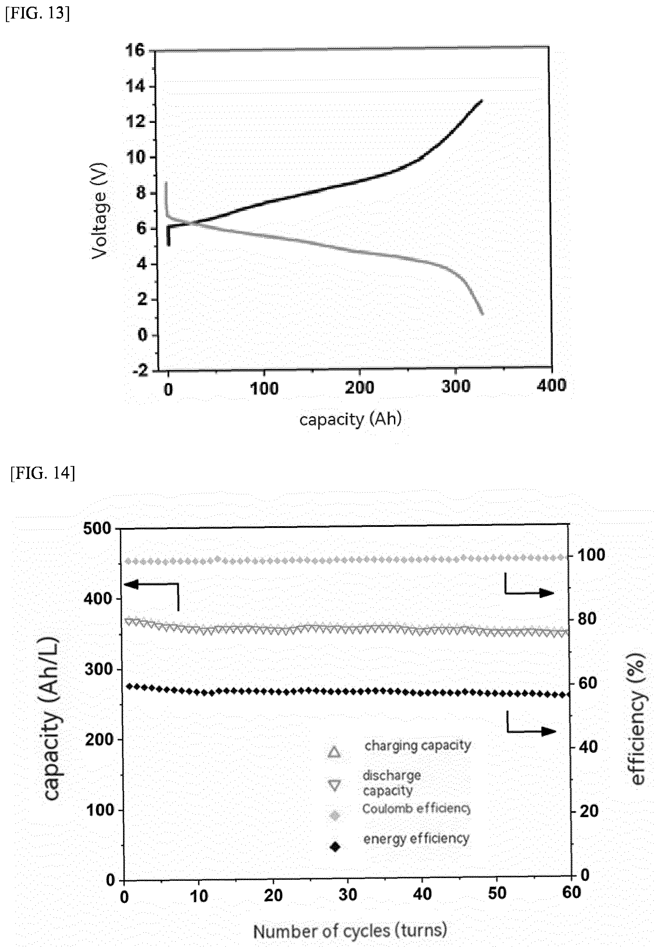

FIG. 13 shows a charge-discharge curve during the operation of the battery stack composed of 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery and the vanadium negative electrode electrolyte. The test conditions are: the current density is 60.92 mA cm⁻², and the cut-off voltages are 13 V and 1 V.
FIG. 14 shows a cycle stability test of the battery stack (under air conditions) composed of 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery and the vanadium negative electrode electrolyte. The test conditions are: the current density is 60.92 mA cm⁻², and the cut-off voltages are 13 V and 1 V.

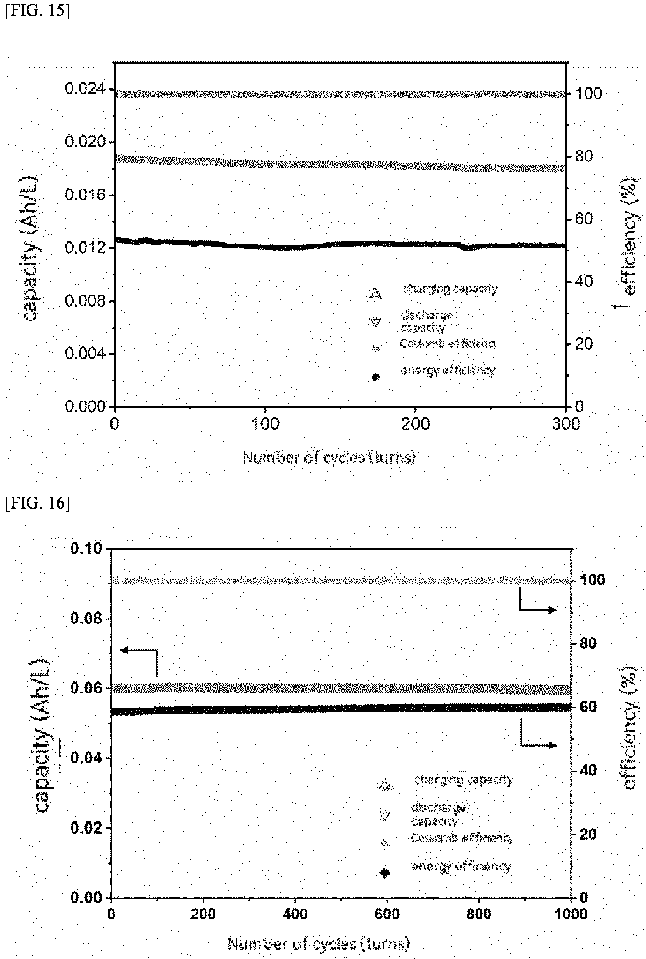

FIG. 15 shows a cycle stability curve of the flow battery long-cycle test (under air conditions) using 3-benzylamine-2-hydroxy-1,4-dicarbonylnaphthalene (naphthoquinone compound) and 1,2,3-trihydroxy-3-benzylaminonaphthalene (naphthol compound) as the positive electrode electrolyte of the flow battery. The test conditions are: the current density is 40 mA cm⁻², and the cut-off voltages are 1.4 V and 0.1 V.
FIG. 16 shows a cycle stability curve of the flow battery long-cycle test (under air conditions) using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery. The test conditions are: the current density is 40 mA cm⁻², and the cut-off voltages are 1.4 V and 0.1 V.

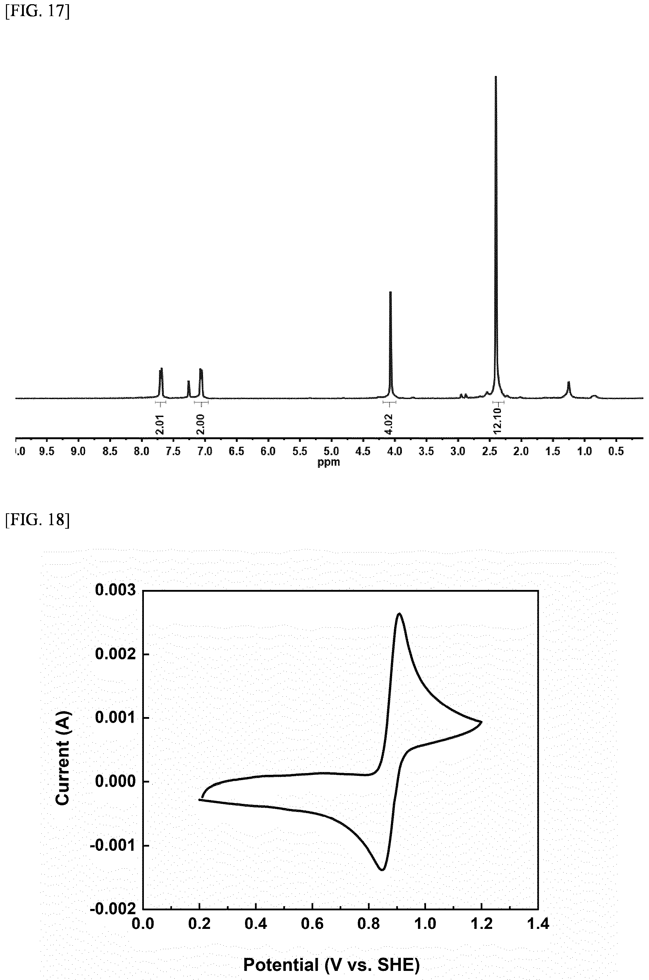

FIG. 17 shows a nuclear magnetic resonance hydrogen spectrum of the product 2,6-dibenzylamine-1,5-dihydroxynaphthalene (naphthol compound) prepared from 2,6-dihydroxynaphthalene in Example 15.
FIG. 18 shows a cyclic voltammetry test curve of Example 15 for preparing 2,6-dibenzylamine-1,5-dicarbonylnaphthalene (naphthoquinone compound) by using 2,6-dibenzylamine-1,5-dihydroxynaphthalene (naphthol compound) as raw material through an electrochemical reaction cell.
   The test conditions are: using graphite as the working electrode and counter electrode, using a saturated calomel electrode as the reference electrode, and the scanning speed is 50 mV/s.

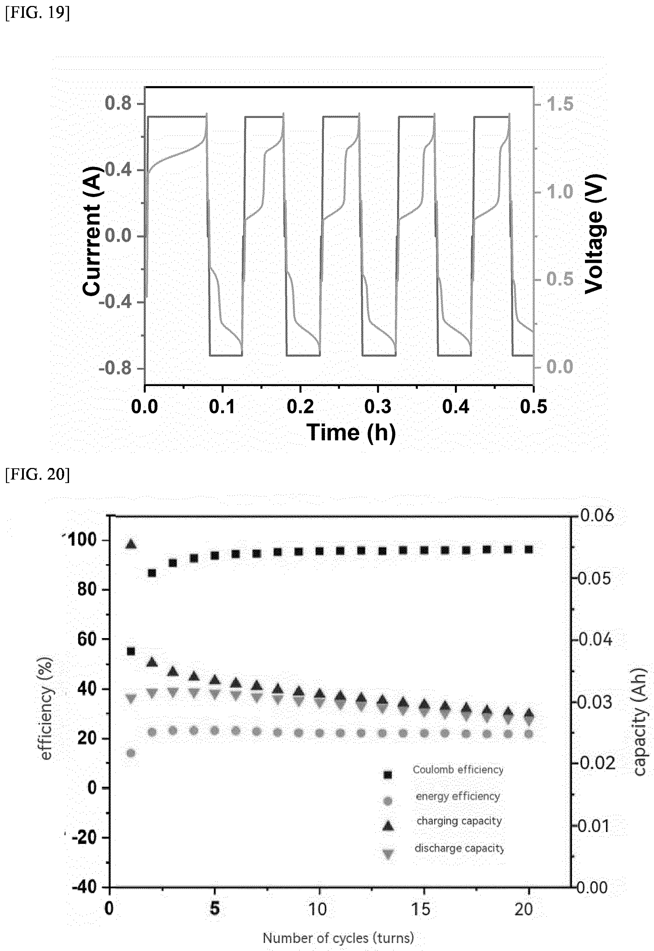

FIG. 19 shows a charge-discharge curve of the electrochemical redox test of the flow battery using 2,6-dibenzylamine-1,5-dicarbonylnaphthalene (naphthoquinone compound) and 2,6-dibenzylamine-1,5-dihydroxynaphthalene (naphthol compound) as the positive electrode electrolyte in Example 15. The test conditions are: the current density is 80 mA cm⁻², and the cut-off voltages are 1.4 V and 0.1 V.
FIG. 20 shows a cycle stability test of the flow battery using 2,6-dibenzylamine-1,5-dicarbonylnaphthalene (naphthoquinone compound) and 2,6-dibenzylamine-1,5-dihydroxynaphthalene (naphthol compound) as the positive electrode electrolyte in Example 15. The test conditions are: the current density is 80 mA cm⁻², and the cut-off voltages are 1.4 V and 0.1 V.

### SPECIFIC IMPLEMENTATIONS

In order to further illustrate the present application, the following examples are listed in conjunction with the experimental results and the drawings, but they do not limit the scope of the invention defined by the claims.

### Example 1

**This Example illustrates** the **preparation process of 3-benzylamine-2-hydroxy-1,4-dicarbonylnaphthalene (naphthoquinone compound) and 1,2,3-trihydroxy-3-benzylaminonaphthalene (naphthol compound)** via **an electrochemical reaction cell.**

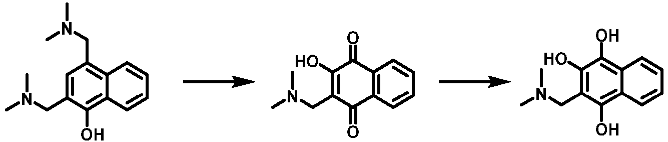

2,4-dibenzylamine-1-naphthol was dissolved in 3 mol/L sulfuric acid to prepare a 0.01 mol/L solution (50 mL). The solution was transferred to an electrochemical reaction cell, using a graphite plate as the working electrode (1 cm²) and counter electrode (4 cm²), and a saturated calomel electrode as the reference electrode. Scanning was performed from low potential to high potential, then from high potential to low potential, with a scanning range of 0 V to 1 V, a scanning rate of 50 mV/s, and 500 scanning cycles. The naphthoquinone compound 3-benzylamine-2-hydroxy-1,4-dicarbonylnaphthalene was obtained at 0.25-0.30 V (vs. SCE), and the naphthol compound 1,2,3-trihydroxy-3-benzylaminonaphthalene was obtained at 0.15-0.2 V (vs. SCE). The products were confirmed by characterization methods such as mass spectrometry, ¹H NMR spectroscopy, and ultraviolet spectroscopy, with a yield of 60%. The water solubility of the raw material under acidic conditions was lower than 5 mM, and the water solubility of the electrochemical oxidation product under acidic conditions was approximately 1 M.

FIG. 1 shows the cyclic voltammetry test curves of Example 1 for preparing 3-benzylamine-2-hydroxy-1,4-dicarbonylnaphthalene (naphthoquinone compound) and 1,2,3-trihydroxy-3-benzylaminonaphthalene (naphthol compound) using 2,4-dibenzylamine-1-naphthol as the raw material through an electrochemical reaction cell. The test conditions were: graphite as the working electrode and counter electrode, saturated calomel electrode as the reference electrode, and a scanning speed of 50 mV/s.

### Example 2

**This example illustrates the preparation process of 3-benzylamine-2-hydroxy-1,4-dicarbonylnaphthalene (naphthoquinone compound) and 1,2,3-trihydroxy-3-benzylaminonaphthalene (naphthol compound) via an aqueous redox battery.**

2,4-Dibenzylamine-1-naphthol was dissolved in 3 mol/L sulfuric acid to prepare a 0.1 mol/L solution (20 mL) as the positive electrode electrolyte, and silicotungstic acid was dissolved in 3 mol/L sulfuric acid to prepare a 0.2 mol/L solution (20 mL) as the negative electrode electrolyte. An aqueous redox battery was assembled with a graphite plate as the current collector, graphite felt as the porous electrode, and polybenzimidazole as the separator. Charging was performed in a constant current mode at a current density of 40 mA cm⁻² until the charging cut-off voltage of 1.4 V, obtaining the naphthoquinone compound 3-benzylamine-2-hydroxy-1,4-dicarbonylnaphthalene. Discharging was continued in a constant current mode at a current density of 40 mA cm⁻² until the discharging cut-off voltage of 0.1 V, obtaining the naphthol compound 1,2,3-trihydroxy-3-benzylaminonaphthalene. The products were confirmed by characterization methods such as mass spectrometry, ¹H NMR spectroscopy, and ultraviolet spectroscopy, with a yield of 72%. The water solubility of the raw material under acidic conditions was lower than 5 mM, and the water solubility of the electrochemical oxidation product under acidic conditions was approximately 1 M.

FIG. 2 shows the physical diagram of the positive electrode electrolyte of Example 2 for preparing 3-benzylamine-2-hydroxy-1,4-dicarbonylnaphthalene (naphthoquinone compound) using 2,4-dibenzylamine-1-naphthol as the raw material through an aqueous redox battery. The left suspension is a 3 mol/L sulfuric acid solution of 0.1 mol/L 2,4-dibenzylamine-1-naphthol, and the right clear solution is a 3 mol/L sulfuric acid solution of the electrochemical oxidation product 3-benzylamine-2-hydroxy-1,4-dicarbonylnaphthalene.

### Example 3

**This Example illustrates the preparation process of 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) via an electrochemical reaction cell.**

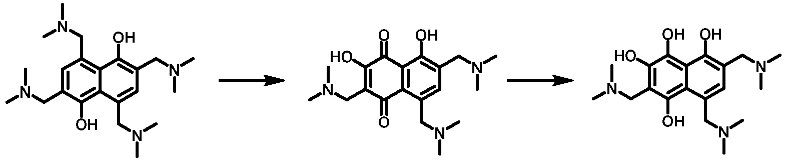

2,4,6,8-Tetrabenzylamine-1,5-naphthalenediol was dissolved in 3 mol/L sulfuric acid to prepare a 0.01 mol/L solution (50 mL). The solution was transferred to an electrochemical reaction cell, using a graphite plate as the working electrode (1 cm²) and counter electrode (4 cm²), and a saturated calomel electrode as the reference electrode. Scanning was performed from low potential to high potential, then from high potential to low potential, with a scanning range of 0 V to 1 V, a scanning rate of 50 mV/s, and 1000 scanning cycles. The naphthoquinone compound 3,5,7-tribenzylamine-2,8-dihydroxy-1,4-dicarbonylnaphthalene was obtained at 0.32-0.35 V (vs. SCE), and the naphthol compound 1,2,4,8-tetrahydroxy-3,5,7-tribenzylaminonaphthalene was obtained at 0.25 V (vs. SCE). The products were confirmed by characterization methods such as mass spectrometry, ¹H NMR spectroscopy, and ultraviolet spectroscopy, with a yield of 70%. The water solubility of the raw material under acidic conditions was lower than 1 M, and the water solubility of the electrochemical oxidation product under acidic conditions was approximately 1.6 M.

FIG. 3 shows the cyclic voltammetry test curves of Example 3 for preparing 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) using 2,4,6,8-tetrabenzylamine-1,5-naphthalenediol as the raw material through an electrochemical reaction cell. The test conditions were: graphite as the working electrode and counter electrode, saturated calomel electrode as the reference electrode, and a scanning speed of 50 mV/s.

### Example 4

**This Example illustrates** the **preparation process of 3,5,7-tribenzylamine-2,8-dihydroxy-1,4-dicarbonylnaphthalene (naphthoquinone compound) and** 1,2,4,8-tetrahydroxy-3,5,7-tribenzylaminonaphthalene **(naphthol compound) via an aqueous redox battery.**

2,4,6,8-Tetrabenzylamine-1,5-naphthalenediol was dissolved in 3 mol/L sulfuric acid to prepare a 0.1 mol/L solution (20 mL) as the positive electrode electrolyte, and silicotungstic acid was dissolved in 3 mol/L sulfuric acid to prepare a 0.2 mol/L solution (20 mL) as the negative electrode electrolyte. An aqueous redox battery was assembled with a graphite plate as the current collector, graphite felt as the porous electrode, and polybenzimidazole as the separator. Charging was performed in a constant current mode at a current density of 40 mA cm⁻² until the charging cut-off voltage of 1.4 V, obtaining the naphthoquinone compound 3,5,7-tribenzylamine-2,8-dihydroxy-1,4-dicarbonylnaphthalene. Discharging was continued in a constant current mode at a current density of 40 mA cm⁻² until the discharging cut-off voltage of 0.1 V, obtaining the naphthol compound 1,2,4,8-tetrahydroxy-3,5,7-tribenzylaminonaphthalene. The products were confirmed by characterization methods such as mass spectrometry, ¹H NMR spectroscopy, and ultraviolet spectroscopy, with a yield of 92%. The water solubility of the raw material under acidic conditions was lower than 1 M, and the water solubility of the electrochemical oxidation product under acidic conditions was approximately 1.6 M.

FIG. 4 shows the characteristic molecular weight of the raw material obtained by mass spectrometry analysis in the process of preparing 3,5,7-tribenzylamine-2,8-dihydroxy-1,4-dicarbonylnaphthalene (naphthoquinone compound) using 2,4,6,8-tetrabenzylamine-1,5-naphthalenediol as the raw material through an aqueous redox battery in Example 4.

FIG. 5 shows the characteristic molecular weight of the electrochemical oxidation product 3,5,7-tribenzylamine-2,8-dihydroxy-1,4-dicarbonylnaphthalene obtained by mass spectrometry analysis in the process of preparing 3,5,7-tribenzylamine-2,8-dihydroxy-1,4-dicarbonylnaphthalene (naphthoquinone compound) using 2,4,6,8-tetrabenzylamine-1,5-naphthalenediol as the raw material through an aqueous redox battery in Example 4.

FIG. 6 shows the characteristic molecular weight of 1,2,4,8-tetrahydroxy-3,5,7-tribenzylaminonaphthalene obtained by mass spectrometry analysis in the process of preparing 1,2,4,8-tetrahydroxy-3,5,7-tribenzylaminonaphthalene (naphthol compound) using 2,4,6,8-tetrabenzylamine-1,5-naphthalenediol as the raw material through an aqueous redox battery in Example 4.

### Example 5

**This Example illustrates the preparation process of 2,2',6,6'-tetrabenzylamine-5,5'-dihydroxy-1,1'-dicarbonylbinaphthalene (naphthoquinone compound) and 1,1',5,5'-tetrahydroxy-2,2',6,6'-tetrabenzylaminonaphthalene (naphthol compound) via an electrochemical reaction cell.**

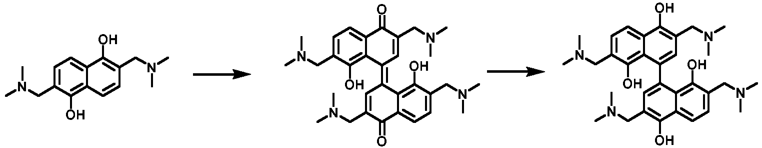

2,6-Dibenzylamine-1,5-naphthalenediol was dissolved in 3 mol/L sulfuric acid to prepare a 0.01 mol/L solution (50 mL). The solution was transferred to an electrochemical reaction cell, using a graphite plate as the working electrode (1 cm²) and counter electrode (4 cm²), and a saturated calomel electrode as the reference electrode. Scanning was performed from low potential to high potential, then from high potential to low potential, with a scanning range of 0 V to 1 V, a scanning rate of 50 mV/s, and 500 scanning cycles. The naphthoquinone compound 2,2',6,6'-tetrabenzylamine-5,5'-dihydroxy-1,1'-dicarbonylbinaphthalene was obtained at 0.23-0.24 V (vs. SCE), and the naphthol compound 1,1',5,5'-tetrahydroxy-2,2',6,6'-tetrabenzylaminonaphthalene was obtained at 0.21-0.22 V (vs. SCE). The products were confirmed by characterization methods such as mass spectrometry, ¹H NMR spectroscopy, and ultraviolet spectroscopy, with a yield of 70%. The water solubility of the raw material under acidic conditions was lower than 5 mM, and the water solubility of the electrochemical oxidation product under acidic conditions was approximately 2 M.

FIG. 7 shows the cyclic voltammetry test curves of Example 5 for preparing 2,2',6,6'-tetrabenzylamine-5,5'-dihydroxy-1,1'-dicarbonylbinaphthalene (naphthoquinone compound) and 1,1',5,5'-tetrahydroxy-2,2',6,6'-tetrabenzylaminonaphthalene (naphthol compound) using 2,6-dibenzylamine-1,5-naphthalenediol as the raw material through an electrochemical reaction cell. The test conditions were: graphite as the working electrode and counter electrode, saturated calomel electrode as the reference electrode, and a scanning speed of 50 mV/s.

### Example 6

**This Example illustrates the preparation process of 2,2',6,6'-tetrabenzylamine-5,5'-dihydroxy-1,1'-dicarbonylbinaphthalene (naphthoquinone compound) and 1,1',5,5'-tetrahydroxy-2,2',6,6'-tetrabenzylaminonaphthalene (naphthol compound) via an aqueous redox battery.**

2,6-Dibenzylamine-1,5-naphthalenediol was dissolved in 3 mol/L sulfuric acid to prepare a 0.1 mol/L solution (20 mL) as the positive electrode electrolyte, and silicotungstic acid was dissolved in 3 mol/L sulfuric acid to prepare a 0.2 mol/L solution (20 mL) as the negative electrode electrolyte. An aqueous redox battery was assembled with a graphite plate as the current collector, graphite felt as the porous electrode, and polybenzimidazole as the separator. Charging was performed in a constant current mode at a current density of 40 mA cm⁻² until the charging cut-off voltage of 1.4 V, obtaining the naphthoquinone compound 2,2',6,6'-tetrabenzylamine-5,5'-dihydroxy-1,1'-dicarbonylbinaphthalene. Discharging was continued in a constant current mode at a current density of 40 mA cm⁻² until the discharging cut-off voltage of 0.1 V, obtaining the naphthol compound 1,1',5,5'-tetrahydroxy-2,2',6,6'-tetrabenzylaminonaphthalene. The products were confirmed by characterization methods such as mass spectrometry, ¹H NMR spectroscopy, and ultraviolet spectroscopy, with a yield of 70%. The water solubility of the raw material under acidic conditions was lower than 5 mM, and the water solubility of the electrochemical oxidation product under acidic conditions was approximately 2 M.

FIG. 8 shows a physical diagram of the positive electrode electrolyte of Example 6 for preparing 2,2',6,6'-tetrabenzylamine-5,5'-dihydroxy-1,1'-dicarbonylbinaphthalene (naphthoquinone compound) using 2,6-dibenzylamine-1,5-naphthalenediol as the raw material through an electrochemical reaction cell. The left suspension is a 3 mol/L sulfuric acid solution of 0.1 mol/L 2,6-dibenzylamine-1,5-naphthalenediol, and the right clear solution is a 3 mol/L sulfuric acid solution of the electrochemical oxidation product 2,2',6,6'-tetrabenzylamine-5,5'-dihydroxy-1,1'-dicarbonylbinaphthalene.

### Example 7

**This Example illustrates the composition of an aqueous redox battery using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode redox couple, and ZnCl₂ and Zn as the negative electrode redox couple.**

3,5,7-tribenzylamine-2,8-dihydroxy-1,4-dicarbonylnaphthalene (naphthoquinone compound) of 0.1 mol/L was used as the positive electrode electrolyte (10 mL), and zinc chloride was prepared into a 0.2 mol/L solution (10 mL) and adjusted to pH=3 with sulfuric acid as the negative electrode electrolyte. An aqueous redox battery was assembled with a graphite plate as the current collector, graphite felt as the porous electrode, and polybenzimidazole as the separator. A zinc foil with a thickness of 0.1 mm and an area of 1 cm² was added to the negative electrode side. Discharging was performed in a constant current mode at a current density of 10 mA cm⁻² until the discharging cut-off voltage of 0.1 V, followed by charging in a constant current mode at a current density of 10 mA cm⁻² until the charging cut-off voltage of 1.2 V. One redox process is a complete charge-discharge cycle, and multiple charge-discharge cycles can be achieved. Such water-soluble naphthalene-type compounds are expected to be applied in aqueous redox batteries.

### Example 8

This Example illustrates the electrochemical redox test conducted using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte for a flow battery.

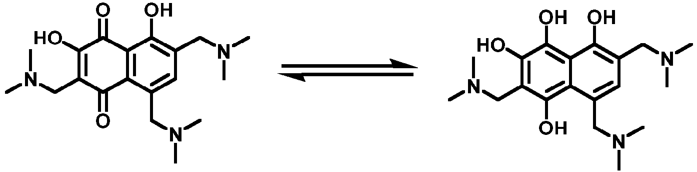

The starting material for the oxidized naphthoquinone and reduced naphthol was 2,4,6,8-tetrabenzylamine-1,5-naphthalenediol, and the target products were obtained via an electrochemical oxidation reaction. The specific procedures were as follows:
A sulfuric acid aqueous solution of 3.0 mol/L was prepared. The naphthol precursor, 2,4,6,8-tetrabenzylamine-1,5-naphthalenediol, was dissolved in the aforementioned sulfuric acid solution to prepare 20 mL of a 0.1 mol/L electrolyte. Subsequently, silicotungstic acid hydrate was dissolved in the same 3.0 mol/L sulfuric acid solution to obtain 80 mL of a 0.1 mol/L silicotungstic acid electrolyte. The resulting 2,4,6,8-tetrabenzylamine-1,5-naphthalenediol and silicotungstic acid electrolytes were reserved for use. A flow battery was assembled using a graphite plate as the current collector, graphite felt as the electrode material, a polybenzimidazole ion-exchange membrane as the separator, and components such as a tetrafluoro flow frame and stainless steel end plates. The 2,4,6,8-tetrabenzylamine-1,5-naphthalenediol and silicotungstic acid electrolytes were employed for the positive and negative electrodes of the flow battery, respectively. A peristaltic pump and latex tubing were used to circulate the electrolytes from their respective storage tanks into the battery cavity and back, ensuring continuous flow and full infiltration of the graphite felt. Subsequently, the assembled battery underwent charge-discharge redox testing under a constant current mode. The charge and discharge currents were both set to 1.92 A, corresponding to a current density of 40 mA cm⁻², with cutoff voltages of 1.4 V and 0.1 V, respectively. The stability of the battery was evaluated through cyclic charge-discharge tests. The current-voltage curve near 330 hours of testing was selected, as shown in FIG. 9, which demonstrated stable charge-discharge operation and consistent capacity retention. The structures of the oxidized and reduced states of the corresponding naphthalene-type compounds were characterized using techniques such as mass spectrometry, nuclear magnetic resonance hydrogen spectroscopy, and ultraviolet spectroscopy.

### Example 9

This Example illustrates the long-cycle electrochemical stability test of a flow battery using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte, paired with a silicotungstic acid negative electrode under low-concentration conditions.

The starting material for the oxidized naphthoquinone and reduced naphthol was 2,4,6,8-tetrabenzylamine-1,5-naphthalenediol, and the target products were obtained through an electrochemical oxidation reaction. The specific operations were as follows:
A sulfuric acid aqueous solution of 3.0 mol/L was prepared. The naphthol precursor, 2,4,6,8-tetrabenzylamine-1,5-naphthalenediol, was dissolved in this sulfuric acid solution to prepare 20 mL of a 0.1 mol/L electrolyte. Separately, silicotungstic acid hydrate was dissolved in the same 3.0 mol/L sulfuric acid solution to prepare 80 mL of a 0.1 mol/L silicotungstic acid electrolyte. These electrolytes were stored for later use. A flow battery was assembled with a graphite plate as the current collector, graphite felt as the electrode material, a polybenzimidazole ion-exchange membrane as the separator, and components including a tetrafluoro flow frame and stainless steel end plates. The 2,4,6,8-tetrabenzylamine-1,5-naphthalenediol and silicotungstic acid electrolytes were used for the positive and negative electrodes of the flow battery, respectively. A peristaltic pump and latex tubing were used to circulate the electrolytes from their storage tanks through the battery cavity and back, ensuring thorough infiltration of the graphite felt. The battery was then subjected to charge-discharge redox testing under a constant current mode. The charge and discharge currents were both set to 1.92 A (current density: 40 mA cm⁻²), with cutoff voltages of 1.4 V and 0.1 V, respectively. The battery's cycle stability was evaluated, and as shown in FIG. 10, the charge-discharge capacity remained stable without significant fluctuations or decay. The Coulombic efficiency of the battery approached 100%, and the energy efficiency remained close to 70% throughout the testing period. The structures of the oxidized and reduced states of the corresponding naphthalene-type compounds were confirmed using characterization techniques such as mass spectrometry, nuclear magnetic resonance hydrogen spectroscopy, and ultraviolet spectroscopy.

### Example 10

This Example illustrates the long-cycle electrochemical stability test of a flow battery using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte, paired with a silicotungstic acid negative electrode under high-concentration conditions.

The starting materials for the oxidized naphthoquinone and reduced naphthol were 2,4,6,8-tetrabenzylamine-1,5-naphthalenediol, and the target products were obtained via an electrochemical oxidation reaction. The specific procedures were as follows:
A sulfuric acid aqueous solution of 3.0 mol/L was prepared. The naphthol precursor, 2,4,6,8-tetrabenzylamine-1,5-naphthalenediol, was dissolved in this sulfuric acid solution to prepare 20 mL of a 1.5 mol/L electrolyte. Separately, silicotungstic acid hydrate was dissolved in the same 3.0 mol/L sulfuric acid solution to prepare 400 mL of a 0.3 mol/L silicotungstic acid electrolyte. These electrolytes were stored for later use. A flow battery was assembled using a graphite plate as the current collector, graphite felt as the electrode material, a polybenzimidazole ion-exchange membrane as the separator, and components such as a tetrafluoro flow frame and stainless steel end plates. The 2,4,6,8-tetrabenzylamine-1,5-naphthalenediol and silicotungstic acid electrolytes were used for the positive and negative electrodes of the flow battery, respectively. A peristaltic pump and latex tubing were used to circulate the electrolytes from their storage tanks through the battery cavity and back, ensuring full infiltration of the graphite felt. The battery was then subjected to charge-discharge redox testing under a constant current mode. The charge and discharge currents were both set to 1.92 A (current density: 40 mA cm⁻²), with cutoff voltages of 1.4 V and 0.1 V, respectively. The battery's cycle stability was evaluated, and as shown in FIG. 11, the charge-discharge capacity remained stable with no significant fluctuations or decay. The Coulombic efficiency of the battery approached 100%, and the average energy efficiency was approximately 66%. The battery exhibited good cycle stability under high-concentration conditions and exposure to air. The structures of the oxidized and reduced states of the corresponding naphthalene-type compounds were confirmed using characterization techniques such as mass spectrometry, nuclear magnetic resonance hydrogen spectroscopy, and ultraviolet spectroscopy.

### Example 11

This Example illustrates the long-cycle electrochemical stability test of using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of a flow battery, paired with a vanadium negative electrode under high-concentration conditions.

The starting material for the oxidized naphthoquinone and reduced naphthol was 2,4,6,8-tetrabenzylamine-1,5-naphthalenediol, and the target products were obtained through an electrochemical oxidation reaction. The specific operations were as follows:
A sulfuric acid aqueous solution of 3.0 mol/L was prepared. The naphthol precursor 2,4,6,8-tetrabenzylamine-1,5-naphthalenediol was dissolved in the above sulfuric acid solution to prepare a 1.0 mol/L positive electrode electrolyte. The negative electrode used a 1 mol/L V³⁺ electrolyte prepared with 3.0 mol/L sulfuric acid. The obtained 2,4,6,8-tetrabenzylamine-1,5-naphthalenediol and V³⁺ electrolytes were set aside. A flow battery was assembled with a graphite plate as the current collector, graphite felt as the electrode material, a polybenzimidazole ion-exchange membrane as the separator, and components such as a tetrafluoro flow frame and stainless steel end plate. The 2,4,6,8-tetrabenzylamine-1,5-naphthalenediol and V³⁺ electrolytes were respectively used for the positive and negative electrodes of the flow battery. A peristaltic pump and latex tube were used to pump the electrolytes from the storage tank into the battery cavity and return them to the storage tank, allowing the electrolytes to circulate in and out and fully infiltrate the graphite felt. Then, the above battery was subjected to charge-discharge redox testing. The charge-discharge current was set to 1.92 A, i.e., a current density of 40 mA cm⁻², with cut-off voltages of 1.4 V and 0.1 V respectively. The battery was subjected to a cycle stability test. As shown in FIG. 12, the battery charge-discharge capacity remained stable without significant capacity fluctuations or decay. The battery Coulombic efficiency was close to 100%, and the average energy efficiency was approximately 72%. The battery showed good cycle stability under high-concentration and air conditions. The structures of the oxidized and reduced states of the corresponding naphthalene-type compounds were confirmed by characterization methods such as mass spectrometry, ¹H NMR spectroscopy, and ultraviolet spectroscopy.

### Example 12

This example illustrates the electrochemical redox and cycle stability test of scaling up the battery using a stack paired with a vanadium negative electrode, using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of the flow battery.

The starting material for the oxidized naphthoquinone and reduced naphthol was 2,4,6,8-tetrabenzylamine-1,5-naphthalenediol, and the target products were obtained through an electrochemical oxidation reaction. The specific operations were as follows:
A sulfuric acid aqueous solution of 3.0 mol/L was prepared. The naphthol precursor 2,4,6,8-tetrabenzylamine-1,5-naphthalenediol was dissolved in the above sulfuric acid solution to prepare 14 L of a 0.6 mol/L positive electrode electrolyte. The negative electrode used a 1 mol/L V³⁺ electrolyte prepared with 3.0 mol/L sulfuric acid. The obtained 2,4,6,8-tetrabenzylamine-1,5-naphthalenediol and V³⁺ electrolytes were set aside. A 10-section 476 cm² stack was assembled with a graphite plate as the current collector, graphite felt as the electrode material, a polybenzimidazole ion-exchange membrane as the separator, and components such as a tetrafluoro flow frame and stainless steel end plate. The 2,4,6,8-tetrabenzylamine-1,5-naphthalenediol and V³⁺ electrolytes were respectively used for the positive and negative electrodes of the flow battery. A magnetic pump and PP pipe were used to pump the electrolytes from the storage tank into the battery cavity and return them to the storage tank, allowing the electrolytes to circulate in and out and fully infiltrate the graphite felt. Then, the above battery was subjected to charge-discharge redox testing. The charge-discharge current was set to 29 A, i.e., a current density of 60.92 mA cm⁻², with cut-off voltages of 13 V and 1 V respectively. The stack was subjected to a cycle stability test, and its charge-discharge curve is shown in FIG. 13. It can be seen from the figure that the battery charge-discharge curve was normal, indicating that the application of naphthalene-type compounds as the positive electrode electrolyte of the flow battery can be scaled up. As shown in FIG. 13, the stack charge-discharge capacity remained stable without significant capacity fluctuations or decay. The battery Coulombic efficiency was close to 100%, and the average energy efficiency was approximately 58%. The stack showed good cycle stability under relatively high concentration and air conditions. The strategy of using naphthalene-type compounds as the positive electrode of the flow battery was successfully scaled up and applied to the flow battery stack test system. The structures of the oxidized and reduced states of the corresponding naphthalene-type compounds were confirmed by characterization methods such as mass spectrometry, ¹H NMR spectroscopy, and ultraviolet spectroscopy.

### Example 13

This Example illustrates the electrochemical redox and cycle stability test of using 3-benzylamine-2-hydroxy-1,4-dicarbonylnaphthalene (naphthoquinone compound) and 1,2,3-trihydroxy-3-benzylaminonaphthalene (naphthol compound) as the positive electrode electrolyte of a flow battery. The reaction equation is as follows:

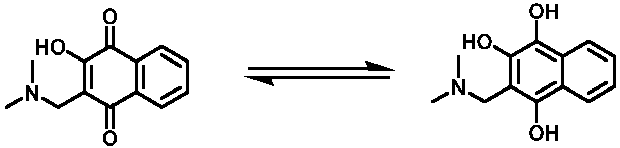

The starting material for the oxidized naphthoquinone and reduced naphthol was 2,4-dibenzylamine-1-naphthol, and the target products were obtained through an electrochemical oxidation reaction. The specific operations were as follows:

A sulfuric acid aqueous solution of 3.0 mol/L was prepared. The naphthol precursor 2,4-dibenzylamine-1-naphthol was dissolved in the above sulfuric acid solution to prepare a 0.1 mol/L electrolyte. Then, silicotungstic acid hydrate was dissolved in the above 3.0 mol/L sulfuric acid solution to obtain a 0.1 mol/L silicotungstic acid electrolyte. The obtained 2,4,6,8-tetrabenzylamine-1,5-naphthalenediol and silicotungstic acid electrolytes were set aside. A flow battery was assembled with a graphite plate as the current collector, graphite felt as the electrode material, a polybenzimidazole ion-exchange membrane as the separator, and components such as a tetrafluoro flow frame and stainless steel end plate. The 2,4-dibenzylamine-1-naphthol and silicotungstic acid electrolytes were respectively used for the positive and negative electrodes of the flow battery. A peristaltic pump and latex tube were used to pump the electrolytes from the storage tank into the battery cavity and return them to the storage tank, allowing the electrolytes to circulate in and out and fully infiltrate the graphite felt. Then, the above battery was subjected to charge-discharge redox testing. The charge-discharge current was set to 1.92 A, i.e., a current density of 40 mA cm⁻², with cut-off voltages of 1.4 V and 0.1 V respectively. The battery stability was such that no significant capacity fluctuations or decay occurred during 300 cycles of charge-discharge redox processes. The battery Coulombic efficiency was close to 100%, and the average energy efficiency reached 52%. The structures of the oxidized and reduced states of the corresponding naphthalene-type compounds were confirmed by characterization methods such as mass spectrometry, ¹H NMR spectroscopy, and ultraviolet spectroscopy.

### Example 14

This Example illustrates the electrochemical redox and cycle stability test of using 3,5,7-tribenzylamine-2,8-dihydroxy-1,4-dicarbonylnaphthalene (naphthoquinone compound) and 1,2,4,8-tetrahydroxy-3,5,7-tribenzylaminonaphthalene (naphthol compound) as the positive electrode electrolyte of a flow battery. The reaction equation is as follows:

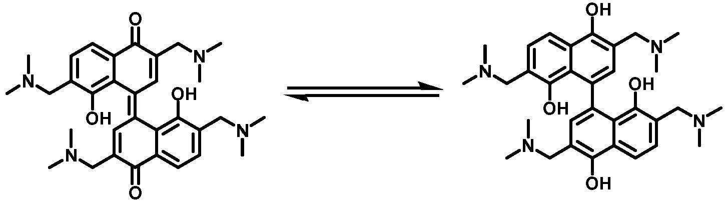

The starting material for the oxidized naphthoquinone and reduced naphthol was 2,6-dibenzylamine-1,5-naphthalenediol, and the target products were obtained through an electrochemical oxidation reaction. The specific operations were as follows:

A sulfuric acid aqueous solution of 3.0 mol/L was prepared. The naphthol precursor 2,6-dibenzylamine-1,5-naphthalenediol was dissolved in the above sulfuric acid solution to prepare a 20 mL electrolyte with a concentration of 0.1 mol/L. Then, silicotungstic acid hydrate was dissolved in the above 3.0 mol/L sulfuric acid solution to obtain 80 mL of 0.1 mol/L silicotungstic acid electrolyte. The obtained 2,4,6,8-tetrabenzylamine-1,5-naphthalenediol and silicotungstic acid electrolytes were set aside. A flow battery was assembled with a graphite plate as the current collector, graphite felt as the electrode material, a polybenzimidazole ion-exchange membrane as the separator, and components such as a tetrafluoro flow frame and stainless steel end plate. The 2,6-dibenzylamine-1,5-naphthalenediol and silicotungstic acid electrolytes were respectively used for the positive and negative electrodes of the flow battery. A peristaltic pump and latex tube were used to pump the electrolytes from the storage tank into the battery cavity and return them to the storage tank, allowing the electrolytes to circulate in and out and fully infiltrate the graphite felt. Then, the above battery was subjected to charge-discharge redox testing. The charge-discharge current was set to 1.92 A, i.e., a current density of 40 mA cm⁻², with cut-off voltages of 1.4 V and 0.1 V respectively. The battery stability was detected by cyclic charge-discharge. No significant capacity fluctuations or decay occurred during 1000 cycles of charge-discharge redox processes. The battery Coulombic efficiency was close to 100%, and the average energy efficiency reached 60%, indicating that the naphthalene-type compounds can achieve stable redox reactions in air and have prospects for large-scale commercialization. The structures of the oxidized and reduced states of the corresponding naphthalene-type compounds were confirmed by characterization methods such as mass spectrometry, ¹H NMR spectroscopy, and ultraviolet spectroscopy.

### Example 15

This Example illustrates the preparation process of 2,6-dibenzylamine-1,5-dicarbonylnaphthalene (naphthoquinone compound) and 2,6-dibenzylamine-1,5-dihydroxynaphthalene (naphthol compound):

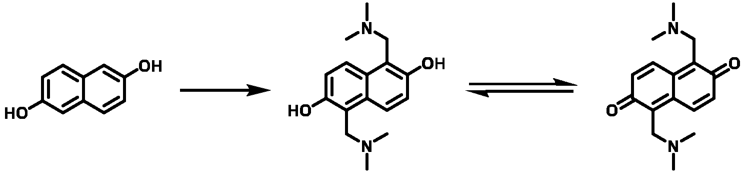

9.5 g of 2,6-dihydroxynaphthalene was dissolved in 100 mL of ethanol. 36 mL of 36% formaldehyde aqueous solution and 70 mL of 40% dimethylamine aqueous solution were added in sequence, and the reaction was carried out at 125°C for 20 hours. After cooling to room temperature, the mixture was filtered and washed with ethanol to obtain white crystals, which were the target product 2,6-dibenzylamine-1,5-dihydroxynaphthalene (naphthol compound). The product was confirmed to be successfully prepared by characterization methods such as mass spectrometry, ¹H NMR spectroscopy, and ultraviolet spectroscopy, with a yield of 86%. Further, 2,6-dibenzylamine-1,5-dihydroxynaphthalene was dissolved in 3 mol/L sulfuric acid to prepare a 0.01 mol/L solution (50 mL). The solution was transferred to an electrochemical reaction cell with a graphite plate as the working electrode (1 cm²) and counter electrode (4 cm²), and a saturated calomel electrode as the reference electrode. Scanning from low potential to high potential generated 2,6-dibenzylamine-1,5-dicarbonylnaphthalene (naphthoquinone compound), with a scanning range of 0 V to 1 V and a scanning rate of 50 mV/s. Scanning from high potential to low potential generated 2,6-dibenzylamine-1,5-dihydroxynaphthalene (naphthol compound), with a scanning range of 1 V to 0 V and a scanning rate of 50 mV/s.

### Example 16

This Example illustrates the electrochemical redox test of using 2,6-dibenzylamine-1,5-dicarbonylnaphthalene (naphthoquinone compound) and 2,6-dibenzylamine-1,5-dihydroxynaphthalene (naphthol compound) as the positive electrode electrolyte of a flow battery. The reaction equation is as follows:

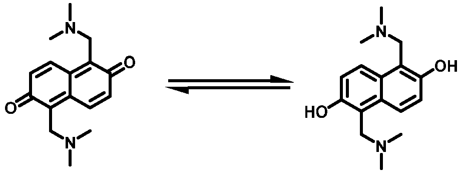

The specific operations were as follows:
A sulfuric acid aqueous solution of 3.0 mol/L was prepared. 2,6-dibenzylamine-1,5-dihydroxynaphthalene was dissolved in the above sulfuric acid solution to prepare 7 mL of a 0.1 mol/L electrolyte. Then, silicotungstic acid hydrate was dissolved in the above 3.0 mol/L sulfuric acid solution to obtain 30 mL of a 0.1 mol/L silicotungstic acid electrolyte. A flow battery was assembled with a graphite plate as the current collector, graphite felt as the electrode material, a polybenzimidazole ion-exchange membrane as the separator, and components such as a tetrafluoro flow frame and stainless steel end plate. The 2,6-dibenzylamine-1,5-dihydroxynaphthalene and silicotungstic acid electrolytes were respectively used for the positive and negative electrodes of the flow battery. A peristaltic pump and latex tube were used to pump the electrolytes from the storage tank into the battery cavity and return them to the storage tank, allowing the electrolytes to circulate in and out and fully infiltrate the graphite felt. Then, the above battery was subjected to charge-discharge redox testing. The charge-discharge current was set to 0.72 A, i.e., a current density of 80 mA cm⁻², with cut-off voltages of 1.4 V and 0.1 V respectively. The assembled battery was subjected to charge-discharge testing, and the current-voltage curve of the battery was recorded, as shown in FIG. 19. During continuous charge-discharge, the battery current-voltage curve was smooth and stable. The structures of the oxidized and reduced states of the corresponding naphthalene-type compounds were confirmed by characterization methods such as mass spectrometry, ¹H NMR spectroscopy, and ultraviolet spectroscopy. The assembled battery was subjected to a cycle stability test, as shown in FIG. 20. During 20 consecutive charge-discharge cycles, the battery Coulombic efficiency reached 96.5%, and the capacity retention rate was 74.2%. The structures of the oxidized and reduced states of the corresponding naphthalene-type compounds were confirmed by characterization methods such as mass spectrometry, ¹H NMR spectroscopy, and ultraviolet spectroscopy.

In addition, the above embodiments are merely some of the embodiments of the present application, and do not limit the present application in any form. Although the present application is disclosed above with the preferred embodiments, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A naphthalene-type compound, **characterized in that**:
the naphthalene-type compound comprises a naphthoquinone compound and/or a naphthol compound;
the naphthoquinone compound has a structure selected from the group consisting of the structures shown in Formula (1), (2), and (3);
the naphthol compound has a structure selected from the group consisting of the structures shown in Formula (4), (5), and (6); 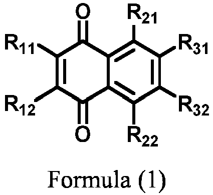 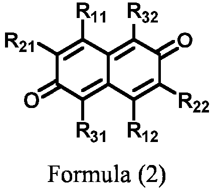 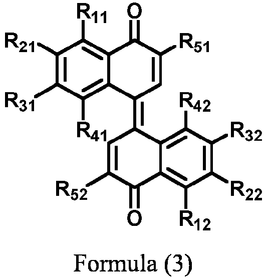 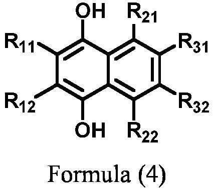 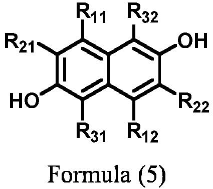 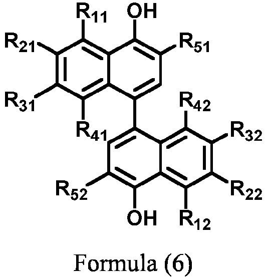
wherein, the naphthalene-type compound having the structure selected from the group consisting of the structures shown in Formula (1), (2), and (3) is an oxidized-state naphthalene-type compound;
the naphthalene-type compound having the structure selected from the group consisted of the structures shown in Formula (4), (5), and (6) is a reduced-state naphthalene-type compound;
the oxidized-state naphthalene-type compound having the structure shown in Formula (1) and the reduced-state naphthalene-type compound having the structure shown in Formula (4) form a redox couple;
the oxidized-state naphthalene-type compound having the structure shown in Formula (2) and the reduced-state naphthalene-type compound having the structure shown in Formula (5) form a redox couple;
the oxidized-state naphthalene-type compound having the structure shown in Formula (3) and the reduced-state naphthalene-type compound having the structure shown in Formula (6) form a redox couple;
R₁₁, R₁₂, R₂₁, R₂₂, R₃₁, R₃₂ are each independently selected from the group consisting of H, F, Cl, Br, I, hydroxyl group, methoxy group, carboxyl group, sulfonic acid group, cyano group, -NR₂, a C₁-C₆ aliphatic group, or a C₁-C₆ aliphatic group substituted with a substituent X-;
the substituent X- in the C₁-C₆ aliphatic group with a substituent X- is selected from the group consisting of carboxyl group, sulfonic acid group, phosphonic acid group, hydroxyl group, methoxy group, or trimethylamino group.

2. A preparation method of the naphthalene-type compound according to claim 1, comprising the following steps:
mixing a substrate with an acid, performing electrochemical charging to obtain the naphthoquinone compound, and continuing electrochemical discharging to obtain the naphthol compound.

3. The preparation method according to claim 2, wherein the substrate has a structure shown in Formula (7): 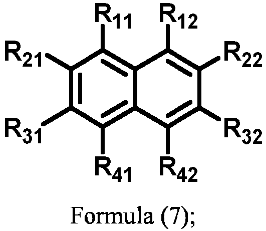
wherein R₁₁, R₂₁, R₃₁, R₄₁, R₁₂, R₂₂, R₃₂, R₄₂ are each independently selected from the group consisting of H, F, Cl, Br, I, hydroxyl group, methoxy group, carboxyl group, sulfonic acid group, cyano group, -NR₂, a C₁-C₆ aliphatic group, or a C₁-C₆ aliphatic group substituted with a substituent X-; and at least one of R₁₁, R₂₁, R₃₁, R₄₁, R₁₂, R₂₂, R₃₂, R₄₂ is a hydroxyl group;
the substituent X- is selected from the group consisting of carboxyl group, sulfonic acid group, phosphonic acid group, hydroxyl group, methoxy group, or trimethylammonio group.

4. The preparation method according to claim 2 or 3, wherein the acid comprises at least one selected from the group consisting of sulfuric acid, hydrochloric acid, phosphoric acid, acetic acid, citric acid, trifluoroacetic acid, and trifluoromethanesulfonic acid;
the concentration of the acid is in a range from 0.1 mol/L to 5 mol/L.

5. The preparation method according to any one of claims 2 to 4, wherein the process of electrochemical charging and electrochemical discharging is carried out in an electrochemical reaction cell or an aqueous battery.

6. The preparation method according to claim 5, wherein
when the process of electrochemical charging and electrochemical discharging is carried out in the electrochemical reaction cell,
a three-electrode system is used with a graphite plate as a working electrode and counter electrode, and a reference electrode selected from the group consisting of a silver-silver chloride electrode, a saturated calomel electrode, and a mercury-mercurous sulfate electrode;
a scan rate is in a range from 5 mV s⁻¹ to 500 mV s⁻¹;
a voltage range is in a range from 0 V to 1 V;
an electrochemical charging oxidation is performed from low voltage to high voltage, followed by an electrochemical discharging reduction from high voltage to low voltage;
a charging voltage range is in a range from 0.23 to 0.35 V (vs. SCE);
a discharging voltage range is in a range from 0.15 to 0.25 V (vs. SCE).

7. The preparation method according to claim 5 or 6, wherein
when the process of electrochemical charging and electrochemical discharging is carried out in the aqueous battery, a constant current mode charging and discharging is adopted;
the substrate and acid side serves as a positive electrode, and a negative electrode contains a conjugated redox compound;
the conjugated redox compound is selected from the group consisting of TiO₂⁺/Ti³⁺, V³⁺/V²⁺ and a polyoxometalate;
the polyoxometalate comprises silicotungstic acid;
a current density is in a range from 1 to 120 mA cm⁻²;
a charging cut-off voltage is in a range from 1.2 to 1.4 V;
a discharging cut-off voltage is in a range from 0 to 0.1 V.

8. An aqueous redox battery, wherein
the aqueous redox battery comprises an electrolyte;
the electrolyte comprises a positive electrolyte and a negative electrolyte;
the positive electrolyte contains the naphthoquinone compound of the naphthalene-type compound according to claim 1 or the naphthoquinone compound prepared by the preparation method according to any one of claims 2 to 7;
and/or
the negative electrolyte contains the naphthol compound of the naphthalene-type compound according to claim 1 or the naphthol compound prepared by the preparation method according to any one of claims 2 to 7.

9. A flow battery, wherein
the flow battery comprises an electrolyte;
the electrolyte comprises a positive electrolyte and a negative electrolyte;
the electrolyte comprises a naphthalene-type compound;
the naphthalene-type compound contains a naphthoquinone compound and/or a naphthol compound;
the positive electrolyte comprises the naphthoquinone compound of the naphthalene-type compound, and the negative electrolyte comprises the naphthol compound of the naphthalene-type compound;
or
the positive electrolyte comprises the naphthol compound of the naphthalene-type compound, and the negative electrolyte comprises the naphthoquinone compound of the naphthalene-type compound;
the concentration of the naphthalene-type compound in the positive electrolyte or negative electrolyte is in a range from 0.01 to 3 mol/L;
wherein the naphthoquinone compound is the naphthoquinone compound of the naphthalene-type compound according to claim 1 or the naphthoquinone compound prepared by the preparation method according to any one of claims 2 to 7;
the naphthol compound is the naphthol compound of the naphthalene-type compound according to claim 1 or the naphthol compound prepared by the preparation method according to any one of claims 2 to 7.

10. The flow battery according to claim 9, wherein
the concentration of the naphthalene-type compound in the positive electrolyte or negative electrolyte is in a range from 1.0 mol/L to 1.5 mol/L.

11. The flow battery according to claim 9 or 10, wherein
the positive electrolyte or negative electrolyte further contains a conjugated redox compound;
the conjugated redox compound is at least one selected from the group consisting of TiO₂⁺/Ti³⁺, V³⁺/V²⁺, and a polyoxometalate;
the polyoxometalate comprises silicotungstic acid.

12. The flow battery according to any one of claims 9 to 11, wherein
the electrolyte further contains an acid;
the acid comprises at least one selected from the group consisting of sulfuric acid, hydrochloric acid, phosphoric acid, acetic acid, citric acid, trifluoroacetic acid, and trifluoromethanesulfonic acid;
the concentration of the acid is in a range from 0.01 mol/L to 6 mol/L.
